# EUROPEAN PATENT SPECIFICATION

(11) **EP 3 727 536 B1**
(45) Date of publication and mention of the grant of the patent: **05.10.2022**
(21) Application number: 18819134.0
(22) Date of filing: 19.12.2018
(51) Int. Cl.: A61M 16/00, A61M 16/14, A61M 11/00, A61M 15/00

(54) **CONTROL UNIT FOR USE WITH A RESPIRATORY ASSIST DEVICE**
STEUEREINHEIT ZUR VERWENDUNG MIT EINER ATEMHILFEVORRICHTUNG
UNITÉ DE COMMANDE À UTILISER AVEC UN DISPOSITIF D'ASSISTANCE RESPIRATOIRE

(30) Priority: 20.12.2017 EP 17208888; 20.12.2017 US 201762608075 P; 31.01.2018 EP 18154322
(43) Date of publication of application: 28.10.2020
(73) Proprietor: Koninklijke Philips N.V., 5656 AG Eindhoven (NL)
(72) Inventor: MARGARIA, Elizabeth Powell, 5656 AE Eindhoven (NL); VON HOLLEN, Dirk Ernest, 5656 AE Eindhoven (NL); SCARBERRY, Eugene Nelson, 5656 AE Eindhoven (NL)
(74) Representative: Philips Intellectual Property & Standards
(86) International application number: PCT/EP2018/085713
(87) International publication number: WO 2019/121830

(56) References cited:
- WO-A1-2011/083468
- US-A1- 2007 023 044
- US-A1- 2014 290 646

## Description

### FIELD OF THE INVENTION

The invention relates to a control unit for use with a respiratory assist device that assists a subject with respiration.

### BACKGROUND OF THE INVENTION

Some illnesses, such as chronic obstructive pulmonary disease (COPD), cause the worst symptoms for subjects while the subject is sleeping and when the subject first wakes up. In the case of COPD, these symptoms (which include dyspnea, coughing, and sputum production) can greatly impact activities of daily living. One of the primary ways to manage the symptoms of an illness or a disease is through inhaled therapies, such as inhaled bronchodilators, corticosteroids, combination therapies, and muscarinic agents.

These inhaled therapies are helpful to subjects. However, they require manual activation, most take a few hours (e.g. 1-2 hours for bronchodilators) for their effectiveness to peak, and they need to be administration regularly (e.g. every 4 hours for bronchodilators) to provide effective relief. It is also often the case that inhalable therapies are missed when a subject is asleep as the interruption to deliver the inhalable therapy is considered a nuisance to the subject and will result in the subject not feeling rested.

There is thus a need for an improved manner in which to deliver an inhalable therapy to a subject, which overcomes these existing problems.

### SUMMARY OF THE INVENTION

As noted above, a limitation with existing inhalable therapy techniques is that the inhalable therapy is often missed while the subject is asleep so as not to interrupt the subject. However, this can have a negative impact on the subject as their symptoms may be worse when they wake up as a result of missing the inhalable therapy while they are asleep. US2007023044 provides for the release of drug based on a patient's sleep stage but it fails to define a manual triggering of the therapy.

It would thus be valuable to have an improved manner in which to deliver an inhalable therapy to a subject, which overcomes the existing problems.

Therefore, according to the invention , there is provided a control unit for use with a respiratory assist device that assists a subject with respiration. The control unit is configured to detect a time at which delivery of an inhalable therapy to the subject is manually triggered on an inhalable therapy device, said inhalable therapy device comprising, a nebulizer, a single dose inhaler, or a metered dose inhaler

. The control unit is also configured to detect whether the subject has fallen asleep and, on detecting that the subject has fallen asleep, control the inhalable therapy device to automatically trigger delivery of the inhalable therapy to the subject at least a predefined length of time following the detected time at which delivery of the inhalable therapy to the subject is manually triggered.

In some embodiments, the control unit may be configured to control the inhalable therapy device to automatically trigger delivery of the inhalable therapy to the subject provided that the subject is detected to still be asleep.

In some embodiments, the control unit may be configured to control the inhalable therapy device to automatically trigger delivery of the inhalable therapy to the subject on expiry of the predefined length of time.

In some embodiments, the control unit may be configured to detect a sleep stage of the subject and control the inhalable therapy device to automatically trigger delivery of the inhalable therapy to the subject on expiry of the predefined length of time provided that the subject is detected to be in a predefined sleep stage.

In some embodiments, the control unit may be further configured to detect a sleep stage of the subject and control the inhalable therapy device to automatically trigger delivery of the inhalable therapy to the subject at a time when the subject is detected to be in a predefined sleep stage after expiry of the predefined length of time. In some embodiments, the control unit may be configured to detect the sleep stage of the subject from physiological characteristic data and/or eye movement data acquired from the subject. In some embodiments, the predefined sleep stage may be a rapid eye movement sleep stage.

In some embodiments, the control unit may be configured to detect whether the subject has fallen asleep based on a breathing pattern of the subject detected by the respiratory assist device.

In some embodiments, the control unit may be configured to control the inhalable therapy device to automatically trigger delivery of the inhalable therapy to the subject at predefined time intervals that begin at least the predefined length of time following the detected time at which delivery of the inhalable therapy to the subject is manually triggered.

In some embodiments, the control unit may be configured to control the inhalable therapy device to further automatically trigger delivery of the inhalable therapy to the subject at a predefined time prior to any one or more of: an estimated wake up time for the subject and an estimated time for an onset of one or more symptoms of the subject.

In some embodiments, the inhalable therapy may comprise any one or more of a medicament, a saline therapy, and a humidity therapy.

In some embodiments, the respiratory assist device may comprise a continuous positive airway pressure device, a non-invasive ventilation device, or an oxygen delivery device.

According to a second aspect, not being part of the present invention , there is provided a method of operating a control unit for use with a respiratory assist device that assists a subject with respiration. The method comprises detecting a time at which delivery of an inhalable therapy to the subject is manually triggered on an inhalable therapy device. The method also comprises detecting whether the subject has fallen asleep and, on detecting that the subject has fallen asleep, controlling the inhalable therapy device to automatically trigger delivery of the inhalable therapy to the subject at least a predefined length of time following the detected time at which delivery of the inhalable therapy to the subject is manually triggered.

According to a third aspect, not being part of the present invention , there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing inhalable therapy techniques are addressed. In particular, according to the above-described aspects and embodiments, the automatic delivery of the inhalable therapy to a subject when the subject is asleep avoids the inhalable therapy having to be missed. The subject (or any other person) does not need to be disturbed to manually deliver the inhalable therapy and thus the burden on the subject is eased and the subject remains rested. Moreover, the symptoms of the subject will be relieved throughout the time period during which the subject is asleep, which means that the symptoms will not awaken the subject and the subject will also feel better on wake up.

Also, it is possible to determine the optimum time for delivery of the inhalable therapy to the subject while the subject is sleeping by detecting the time at which delivery of the inhalable therapy was manually triggered prior to the subject falling asleep and using this to determine the appropriate time to automatically trigger delivery of the inhalable therapy during sleep. Moreover, as the control unit is for use with a respiratory assist device, delivery of the inhalable therapy can be combined with respiratory assistance, which may already be being provided to the subject. The subject can thus benefit from unobtrusive inhalable therapy while asleep, which may improve sleep quality. In this way, the subject will feel more rested and will also be healthier to live a more active life.

There is thus provided an improved manner in which to deliver an inhalable therapy to a subject, which overcomes the existing problems.

These and other aspects will be apparent from and elucidated with reference to the embodiment(s) described hereinafter.

The present invention is defined by the attached claims.

### BRIEF DESCRIPTION OF THE DRAWINGS

Exemplary embodiments will now be described, by way of example only, with reference to the following drawings, in which:
Fig. 1 is a block diagram of a control unit according to an embodiment;
Fig. 2 is a block diagram of a control unit in use in a system according to an example embodiment;
Fig. 3 is a flow chart illustrating a method of operating a control unit according to an embodiment not being part of the present invention;
Fig. 4 is a graphical illustration of sleep stages of a subject according to an example and
Fig. 5 is a flow chart illustrating a method of operating a control unit according to an example embodiment not being part of the present invention.

### DETAILED DESCRIPTION OF EMBODIMENTS

As noted above, there is provided an improved manner in which to deliver an inhalable therapy to a subject, which overcomes existing problems. In particular, there is provided a control unit for use with a respiratory assist device that assists a subject with respiration. The control unit is configured to control an inhalable therapy device to deliver (for example, administer or provide) an inhalable therapy to a subject. The subject may, for example, be a patient.

The control unit can be implemented in numerous ways, with software and/or hardware, to perform the various functions described herein. In particular implementations, the control unit can comprise a plurality of software and/or hardware modules, each configured to perform, or that are for performing, individual or multiple steps of the method described herein. The control unit may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The control unit may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, controllers, DSPs and associated circuitry) to perform other functions.

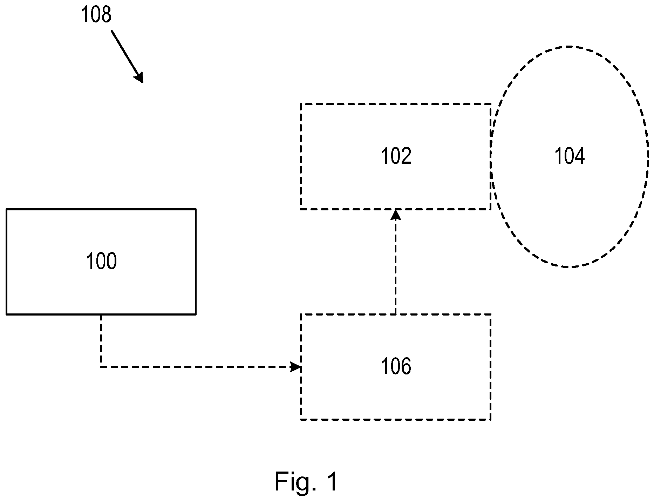

Fig. 1 illustrates an example of a control unit 100 for use with a respiratory assist device 102 that assists a subject 104 with respiration. The respiratory assist device 102 can be any device that is suitable to assist a subject 104 with respiration, either directly or indirectly. Examples of a respiratory assist device 102 include, but are not limited to, a continuous positive airway pressure (CPAP) device, a non-invasive ventilation device, an oxygen delivery device (such as a device for delivering oxygen via a concentrator, cannula, and/or container), or any other device suitable to assist a subject 104 with respiration. Although examples have been provided for the type of respiratory assist device 102, a person skilled in the art will be aware of other types of respiratory assist device 102 with which the control unit 100 may be used.

As mentioned earlier, the control unit 100 is configured to control an inhalable therapy device 106 to deliver an inhalable therapy to the subject 104. The inhalable therapy device 106 comprises a nebulizer, a single dose inhaler, or metered dose inhaler such as a pressurized metered dose inhaler (pMDI). A nebulizer can, for example, be a pneumatic nebulizer, a vibrating mesh nebulizer, an ultrasonic nebulizer, or any other type of nebulizer. An inhaler can, for example, be a dry powder inhaler (dpi), a soft mist inhaler (smi), or any other type of inhaler. The inhalable therapy referred to herein can, for example, comprise any one or more of a medicament (or drug), a saline therapy, a humidity therapy, or any other type of inhalable therapy, or any combination of inhalable therapies. Although examples have been provided for the type of inhalable therapy device 106 and the type of inhalable therapy, a person skilled in the art will be aware of other types of inhalable therapy device 106 that the control unit 100 may be configured to control and other types of inhalable therapy.

As illustrated in Fig. 1, the control unit 100 can be part of a system 108, which may also comprise one or more of the respiratory assist device 102 and the inhalable therapy device 106. Thus, a system 108 may comprise the control unit 100 and that system 108 may also comprise one or more of the respiratory assist device 102 and the inhalable therapy device 106. In the example of the control unit 100 illustrated in Fig. 1, the control unit 100 is illustrated as being external to (i.e. separate to or remote from) the respiratory assist device 102 and the inhalable therapy device 106. Thus, in some embodiments, the control unit 100 may be a separate entity or part of another device (not illustrated).

However, although not illustrated in Fig. 1, the inhalable therapy device 106 may comprise the control unit 100 according to other embodiments or the respiratory assist device 102 may comprise the control unit 100 according to yet other embodiments. For example, the control unit 100 may be attached to or built (or integrated) into the respiratory assist device 102 or the inhalable therapy device 106. In some embodiments where the respiratory assist device 102 comprises the control unit 100, the respiratory assist device 102 may thus control (for example, coordinate and activate) the inhalable therapy device 106 by way of the control unit 100 to deliver the inhalable therapy to a subject 104.

In some embodiments, the inhalable therapy device 106 may be configured to deliver the inhalable therapy directly to the subject 104. For example, the inhalable therapy device 106 may comprise an interface (such as a mask, a cannula, a mouthpiece and/or nose piece, or any other type of interface) configured to be worn over one or more of the nose and mouth of the subject 104 and the inhalable therapy may be delivered to the subject 104 via that interface. Alternatively, in other embodiments, the inhalable therapy device 106 may be configured to deliver the inhalable therapy indirectly to the subject 104. For example, in some embodiments, the inhalable therapy device 106 may be configured to deliver the inhalable therapy to the subject 104 via the respiratory assist device 102. In these embodiments, the respiratory assist device 102 may, for example, comprise an interface (such as a mask, a cannula, a mouthpiece and/or nose piece, or any other type of interface) configured to be worn over one or more of the nose and mouth of the subject 104 and the inhalable therapy may be delivered to the subject 104 via that interface.

Briefly, the control unit 100 described herein is configured to detect a time at which delivery of an inhalable therapy to the subject 104 is manually triggered on an inhalable therapy device 106. The control unit 100 is also configured to detect whether the subject 104 has fallen asleep and, on detecting that the subject 104 has fallen asleep, control the inhalable therapy device 106 to automatically trigger delivery of the inhalable therapy to the subject 104 at least a predefined length of time following the detected time at which delivery of the inhalable therapy to the subject 104 is manually triggered.

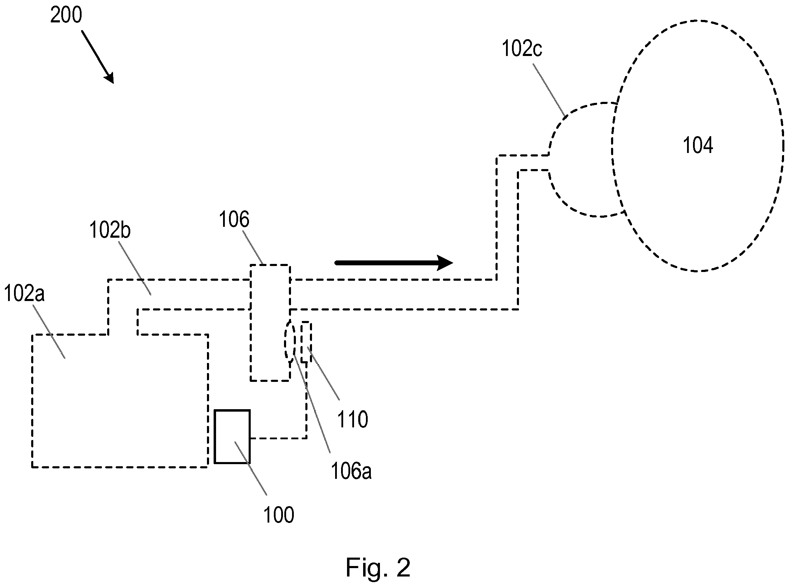

Fig. 2 illustrates an example of the control unit 100 in use in a system 200 according to an embodiment. As mentioned earlier, the control unit 100 is for use with a respiratory assist device 102 that assists a subject 104 with respiration. In this illustrated example embodiment, the respiratory assist device 102 comprises a flow or pressure source 102a (such as a continuous positive airway pressure CPAP source, an oxygen concentrator source, or any other type of flow or pressure source), a tube or tubing 102b, and an interface 102c (such as a mask, a cannula, a mouthpiece and/or nose piece, or any other type of interface) configured to be worn over the nose and/or mouth of the subject 104. The flow or pressure source 102a of the respiratory assist device 102 can be configured to deliver a gas (such as air) to the subject 104 via the tube or tubing 102b and the interface 102c of the respiratory assist device 102. The gas may be delivered by flow or pressure therapy. The control unit 100 is configured to control an inhalable therapy device 106 to deliver an inhalable therapy to the subject 104 in the manner described earlier.

In the example embodiment illustrated in Fig. 2, the inhalable therapy device 106 is connected in-line between the flow or pressure source 102a of the respiratory assist device 102 and the interface 102c of the respiratory assist device 102. In effect, the respiratory assist device 102a provides a respiratory circuit and the inhalable therapy device 106 allows administration of the inhalable therapy in line with this respiratory circuit. The inhalable therapy device 106 according to this illustrated example embodiment comprises a button 106a. The control unit 100 is configured to trigger delivery of the inhalable therapy to the subject 104 by controlling an actuator 110 to activate (or actuate) the button 106a of the inhalable therapy device 106.

On activation (or actuation) of the button 106a, the inhalable therapy device 106 is configured to deliver the inhalable therapy to the subject 104. In this illustrated example embodiment, the inhalable therapy is delivered to the subject 104 via the tube or tubing 102b and the interface 102c of the respiratory assist device 102. For example, the inhalable therapy may be provided from the inhalable therapy device 106 into a gas flowing from the flow or pressure source 102a of the respiratory assist device 102, through the tube or tubing 102b of the respiratory assist device 102 and into the respiratory system of the subject 104 via the interface 102c of the respiratory assist device 102.

Although various arrangements have been illustrated and described for the control unit 100, the respiratory assist device 102 and the inhalable therapy device 106, it will be understood that other arrangements are also possible.

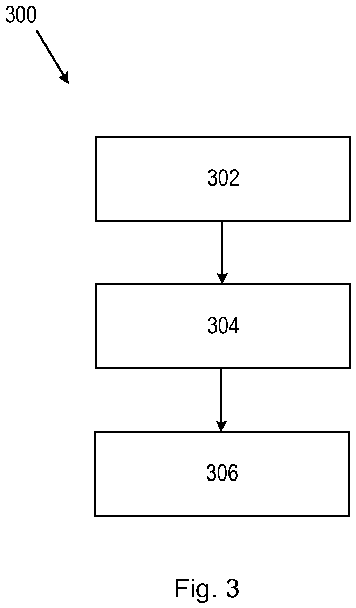

Fig. 3 illustrates a method 300 of operating the control unit 100 described herein according to an embodiment. The method 300 can generally be performed by or under the control of the control unit 100. At block 302, a time at which delivery of an inhalable therapy to the subject 104 is manually triggered on an inhalable therapy device 106 is detected. For example, the inhalable therapy device 106 may comprise a sensor (or detector) and/or circuitry configured to detect the time at which the manual trigger occurs.

In some embodiments, the detected time at which delivery of the inhalable therapy is manually triggered may be stored (or recorded) in a memory of the inhalable therapy device 106. According to some embodiments, the detected time at which delivery of the inhalable therapy is manually triggered may be transmitted from the inhalable therapy device 106 to the respiratory assist device 102. In some embodiments, the inhalable therapy device 106 may be suitable to be docked at the respiratory assist device 102. The inhalable therapy device 106 may be docked at the respiratory assist device 102 such that the inhalable therapy device 106 is in electronic communication with the respiratory assist device 102. In these embodiments, the detected time at which delivery of the inhalable therapy is manually triggered may be transmitted from the inhalable therapy device 106 to the respiratory assist device 102 when the inhalable therapy device 106 is docked at the respiratory assist device 102. A memory or a time register of the memory of the respiratory assist device 102 may then store (or record) the detected time at which delivery of the inhalable therapy is manually triggered.

At block 304, it is detected whether the subject 104 has fallen asleep. For example, it is detected whether sleep onset has begun for the subject 104. In some embodiments, the control unit 100 may be configured to detect whether the subject 104 has fallen asleep based on a breathing pattern of the subject 104. The breathing pattern of the subject 104 is a measure of the respiration of the subject 104 over time (for example, the number of breaths per minute). A breathing pattern is generally irregular when a subject is awake and becomes more uniform when a subject is asleep. Thus, the control unit 100 may be configured to detect that the subject 104 has fallen asleep when the breathing pattern of the subject 104 changes from being irregular to being more uniform. For example, an irregular breathing pattern may involve a greater variation in breath intervals than a more uniform breathing pattern. As such, the control unit 100 may be configured to detect that the subject 104 has fallen asleep when the variation in breath intervals of the subject 104 falls below a predetermined threshold. Alternatively or in addition to the breathing pattern, in some embodiments, the control unit 100 may be configured to detect whether the subject 104 has fallen asleep based on a minute ventilation. For example, the control unit 100 may be configured to detect the subject 104 has fallen asleep when a reduction in minute ventilation is detected. In some of these embodiments, the respiratory assist device 102 may comprise a sensor (or detector) for detecting minute ventilation and, more specifically, a reduction in minute ventilation and the control unit 100 may acquire this information from the respiratory assist device 102.

The control unit 100 may be configured to acquire the breathing pattern of the subject 104 from any device or sensor suitable to detect a breathing pattern of the subject 104. For example, in some embodiments, the breathing pattern of the subject 104 may be detected by the respiratory assist device 102. Alternatively or in addition, in some embodiments, the control unit 100 may be configured to detect that the subject 104 has fallen asleep upon detection of engagement of the respiratory assist device 102 with the subject 104. For example, the respiratory assist device 102 may comprise a sensor (or detector) to detect engagement of the respiratory assist device 102 with the subject 102 and may communicate this information to the control unit 100.

At block 306, on detecting that the subject 104 has fallen asleep, the inhalable therapy device 106 is controlled to automatically trigger delivery of the inhalable therapy to the subject 104 at least a predefined length of time following the detected time at which delivery of the inhalable therapy to the subject 104 is manually triggered. The predefined length of time may be selected based on a length of time the inhalable therapy loses its effectiveness after delivery. More specifically, the predefined length of time may be selected to be less than the length of time for the inhalable therapy to lose its effectiveness after delivery. For example, where an inhalable therapy loses its effectiveness after 8 hours, the predefined length of time may be selected as 4 hours following the detected time at which delivery of the inhalable therapy to the subject 104 is manually triggered.

In some embodiments where the inhalable therapy device 106 is suitable of being docked at the respiratory assist device 102, the respiratory assist device 102 may comprise the control unit 100 that controls the inhalable therapy device 106 to automatically trigger delivery of the inhalable therapy to the subject 104. In this way, the automatic delivery of the inhalable therapy can be delivered from the inhalable therapy device 106 through the respiratory assist device 102 according to some embodiments.

In some embodiments, the control unit 100 may be configured to control the inhalable therapy device 106 to automatically trigger delivery of the inhalable therapy to the subject 104 provided that the subject 104 is detected to still be asleep. In these embodiments, if the control unit 100 detects that the subject is no longer asleep, the control unit 100 may delay automatically triggering delivery of the inhalable therapy to the subject 104. For example, the control unit 100 may delay automatically triggering delivery of the inhalable therapy to the subject 104 until after the control unit 100 detects that the subject 104 has fallen asleep again. Alternatively, the control unit 100 may detect that delivery of the inhalable therapy to the subject 104 is manually triggered on the inhalable therapy device 106 following the detection that the subject is no longer asleep and, in this case, the control unit 100 may be configured to detect whether the subject 104 has fallen asleep again and, on detecting that the subject 104 has fallen asleep again, control the inhalable therapy device 106 to automatically trigger delivery of the inhalable therapy to the subject 104 at least a predefined length of time following this latest detected time at which delivery of the inhalable therapy to the subject 104 is manually triggered.

In some embodiments, the control unit 100 may be configured to control the inhalable therapy device 106 to automatically trigger delivery of the inhalable therapy to the subject 104 on expiry of the predefined length of time. In some of these embodiments, the control unit 100 can be configured to detect a sleep stage of the subject 104 and control the inhalable therapy device 106 to automatically trigger delivery of the inhalable therapy to the subject 104 on expiry of the predefined length of time provided that the subject 104 is detected to be in a predefined sleep stage. Thus, in some embodiments, the control unit 100 can be configured to control the inhalable therapy device 106 to automatically trigger delivery of the inhalable therapy to the subject 104 at the end of the predefined length of time if the subject 104 is in a predefined (or particular) sleep stage at that time. The predefined sleep stage according to these embodiments may, for example, be a rapid eye movement (REM) sleep stage, or any other sleep stage. In some embodiments, a REM sleep stage may be selected as the predefined sleep stage since this is the sleep stage at which an inhalable therapy is least likely to cause arousal of the subject 104 to allow a more restful and better quality sleep for the subject 104. This can be useful, for example, where the inhalable therapy is one that produces side effects that may cause arousal or is a stimulating therapy.

Alternatively to the sleep stage in these embodiments, the control unit 100 may in the same way be configured to detect a circadian rhythm stage or a biological phase of the subject 104 and control the inhalable therapy device 106 to automatically trigger delivery of the inhalable therapy to the subject 104 on expiry of the predefined length of time provided that the subject 104 is detected to be in a predefined circadian rhythm stage or a predefined biological phase.

In other embodiments, the control unit 100 may be configured to detect a sleep stage of the subject 104 and control the inhalable therapy device 106 to automatically trigger delivery of the inhalable therapy to the subject 104 at a time when the subject 104 is detected to be in a predefined sleep stage after expiry of the predefined length of time. For example, the subject 104 may not be in a predefined (or particular) sleep stage at the end of the predefined length of time and thus, in these embodiments, the control unit 100 can be configured to automatically trigger delivery of the inhalable therapy at a later time when the subject 104 is in fact detected to be in the predefined sleep stage. The predefined sleep stage according to these embodiments may, for example, be a rapid eye movement (REM) sleep stage, or any other sleep stage.

Alternatively to the sleep stage in these embodiments, the control unit 100 may in the same way be configured to detect a circadian rhythm stage or a biological phase of the subject 104 and control the inhalable therapy device 106 to automatically trigger delivery of the inhalable therapy to the subject 104 at a time when the subject 104 is detected to be in a predefined circadian rhythm stage or a predefined biological phase after expiry of the predefined length of time.

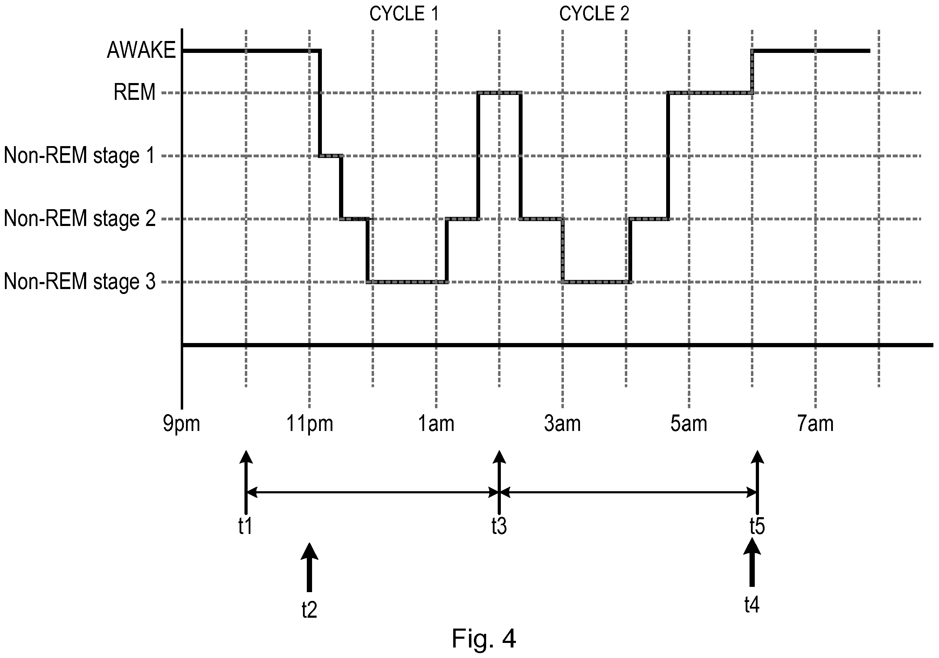

Fig. 4 is a graphical illustration of sleep stages of a subject 104 according to an example embodiment. The sleep stages of the subject 104 in this illustrated example embodiment include a non-rapid eye movement (non-REM) sleep stage 1, a non-rapid eye movement (non-REM) sleep stage 2, a non-rapid eye movement (non-REM) sleep stage 3, and a rapid eye movement (REM) sleep stage. As shown in Fig. 4, the sleep stages of the subject 104 according to this example embodiment occur in two cycles, cycle 1 and cycle 2. In cycle 1, the subject 104 moves from being awake to falling sleep, and then moves to the non-REM sleep stage 1, the non-REM sleep stage 2, the non-REM sleep stage 3, the non-REM sleep stage 2, and the REM sleep stage. In cycle 2, the subject 104 moves from the REM sleep stage to the non-REM sleep stage 2, the non-REM sleep stage 3, the non-REM sleep stage 2, and the REM sleep stage, before waking up.

At a first time t1, delivery of an inhalable therapy to the subject 104 is manually triggered on an inhalable therapy device 106. The subject 104 themselves, a medical professional (such as a doctor), a caregiver, a family member, or any other user may manually trigger the delivery of the inhalable therapy. As described earlier, the control unit 100 detects this time t1 at which delivery of the inhalable therapy to the subject 104 is manually triggered. At a second time t2, the subject 104 goes to bed. In some embodiments, the subject 104 may go to bed using a respiratory assist device 102. The control unit 100 subsequently detects that the subject 104 has fallen asleep, as described earlier. On detecting that the subject 104 has fallen asleep, at a third time t3, the control unit 100 controls an inhalable therapy device 106 to automatically trigger delivery of the inhalable therapy to the subject 104 at least a predefined length of time following the detected time at which delivery of the inhalable therapy to the subject 104 is manually triggered in the manner described earlier. In embodiments where the subject 104 goes to bed using a respiratory assist device 102, the inhalable therapy device 106 may deliver the inhalable therapy to the subject 104 via the respiratory assist device 102.

In the example embodiment illustrated in Fig. 4, the control unit 100 is also configured to detect a sleep stage of the subject 104 and controls the inhalable therapy device 106 to automatically trigger delivery of the inhalable therapy to the subject 104 at a time when the subject 104 is detected to be in a predefined (or specific) sleep stage after expiry of the predefined length of time. Thus, auto-administration of the inhalable therapy to the subject 104 is triggered at time that is greater than the predefined length of time and when it is detected that the subject 104 is in the predefined sleep stage. The predefined sleep stage according to the example embodiment illustrated in Fig. 4 is the rapid eye movement (REM) sleep stage.

At a fourth time t4, the subject 104 wakes up. In embodiments where the subject 104 goes to bed using a respiratory assist device 102, the subject 104 may disconnect from the respiratory assist device 102 following wake-up. At a fifth time t5, delivery of the inhalable therapy to the subject 104 is again manually triggered on the inhalable therapy device 106. The subject 104 themselves, a medical professional (such as a doctor), a caregiver, a family member, or any other user may manually trigger the delivery of the inhalable therapy.

In any of the embodiments described herein where the control unit 100 is configured to detect a sleep stage of the subject 104, the control unit 100 may be configured to detect the sleep stage of the subject 104 from physiological characteristic (or vital sign) data acquired from the subject 104. The physiological characteristic data can be acquired from the subject 104 by one or more physiological characteristic (or vital sign) sensors (not illustrated). In some embodiments, for example, a physiological characteristic sensor can comprise any one or more of a heart rate sensor (or a heart rate variability sensor) configured to detect a heart rate (or heart rate variability) of the subject 104, a respiratory rate sensor configured to detect a respiration rate of the subject 104, a muscle activity sensor configured to detect a muscle activity of the subject 104, a brain activity sensor configured to detect a brain activity of the subject 104, or any other physiological characteristic sensor, or any combination of physiological characteristic sensors.

A heart rate sensor (or heart rate variability sensor) may comprise any sensor suitable to acquire heart rate (or heart rate variability) data from the subject 104, which can be used to detect the sleep stage of the subject 104. Examples of a heart rate sensor (or a heart rate variability sensor) include, but are not limited to, an electrocardiogram (ECG or EKG) sensor, a photoplethysmography (PPG) sensor, a phonocardiography (PCG) sensor, an acceleration sensor, a Doppler radar sensor, or any other heart rate sensor, or any combination of heart rate sensors. A respiratory rate sensor may comprise any sensor suitable to acquire respiratory rate data from the subject 104, which can be used to detect the sleep stage of the subject 104. Examples of a respiratory rate sensor include, but are not limited to, an impedance sensor, an acceleration sensor, a heart rate sensor for detecting heart rate from which respiratory rate can be acquired (such as an electrocardiogram (ECG or EKG) sensor, or a photoplethysmography (PPG) sensor), a Doppler radar sensor, a flow sensor, or any other respiratory rate sensor, or any combination of respiratory rate sensors.

A muscle activity sensor may comprise any sensor suitable to acquire muscle activity data from the subject 104, which can be used to detect the sleep stage of the subject 104. Examples of a muscle activity sensor include, but are not limited to, electromyography (EMG) sensor, an acceleration sensor, or any other muscle activity sensor, or any combination of muscle activity sensors. A brain activity sensor may comprise any sensor suitable to acquire brain activity data from the subject 104, which can be used to detect the sleep stage of the subject 104. Examples of a brain activity sensor include, but are not limited to, an electroencephalography (EEG) sensor, a functional near infra-red (fNIR) sensor, or any other brain activity sensor, or any combination of brain activity sensors.

In any of the embodiments described herein where the control unit 100 is configured to detect a sleep stage of the subject 104, the control unit 100 may be configured to detect the sleep stage of the subject 104 from eye movement data acquired from the subject 104. The eye movement data may be used in addition or alternatively to physiological characteristic (or vital sign) data to detect the sleep stage of the subject 104. The eye movement data can be acquired from the subject 104 by one or more eye movement sensors (not illustrated). An eye movement sensor may comprise any sensor suitable to acquire eye movement data on the subject 104, which can be used to detect the sleep stage of the subject 104. For example, an eye movement sensor may comprise an electrooculography (EOG) sensor, or any other eye movement sensor, or any combination of eye movement sensors.

In embodiments where the control unit 100 is used with (for example, connected to) a respiratory assist device 102, the respiratory assist device 102 may comprise one or more physiological characteristic sensors and/or one or more eye movement sensors. Alternatively or in addition, a wearable device that is configured to be worn by the subject may comprise one or more physiological characteristic sensors and/or one or more eye movement sensors. Alternatively or in addition, one or more physiological characteristic sensors and/or one or more eye movement sensors may be configured to be placed directly onto the subject.

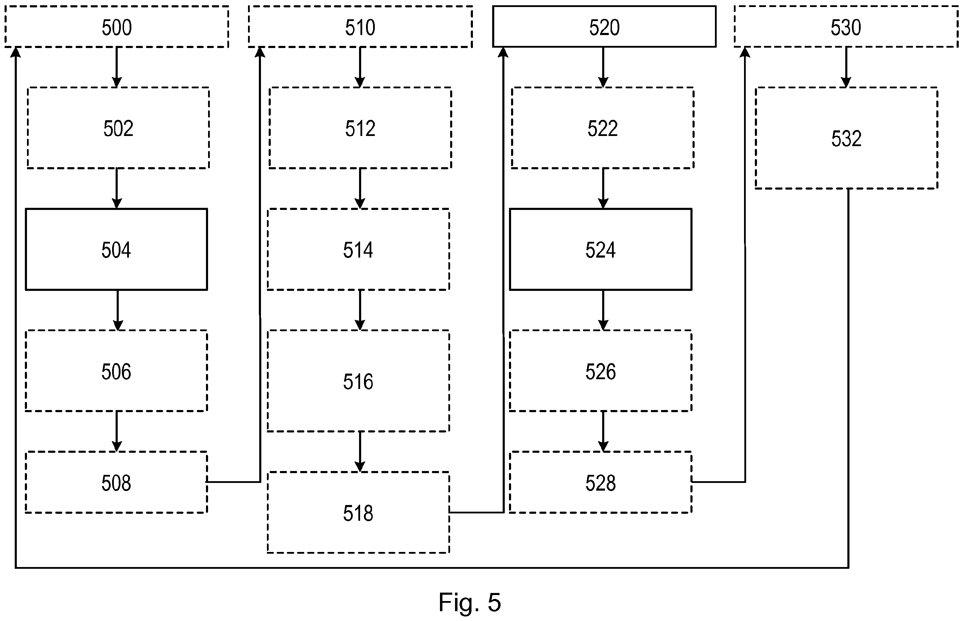

Fig. 5 is a flow chart illustrating a method of operating a control unit 100 according to an example embodiment. At block 500, the subject 104 is awake. At block 502, an inhalable therapy may be prepared in an inhalable therapy device 106. At block 504, delivery of the inhalable therapy to the subject 104 is manually triggered on the inhalable therapy device 106. The subject 104 themselves, a medical professional (such as a doctor), a caregiver, a family member, or any other user may prepare the inhalable therapy device 106 and/or manually trigger the delivery of the inhalable therapy. As described earlier, the control unit 100 detects the time at which delivery of the inhalable therapy to the subject 104 is manually triggered. The manual trigger starts the delivery of the inhalable therapy. At block 506, the inhalable therapy device 106 may thus deliver the inhalable therapy to the subject 104, either directly or indirectly. In the embodiment illustrated in Fig. 2, for example, the inhalable therapy device 106 may deliver the inhalable therapy indirectly to the subject 104 via an interface 102c of a respiratory assist device 102. Alternatively, the inhalable therapy device 106 may itself comprise such an interface to deliver the inhalable therapy directly to the subject 104.

At block 508, the inhalable therapy device 106 may detect a low level of inhalable therapy and automatically stop delivering the inhalable therapy to the subject 104. Alternatively, at block 508, the control unit 100 may detect a low level of inhalable therapy in the inhalable therapy device 106 and control the inhalable therapy device 106 to stop delivering the inhalable therapy to the subject 104. In some embodiments, the control unit 100 may determine a prescribed time of administration of inhalable therapy in order to turn off the inhalable therapy device 106 after the prescribed time. This may leave a predetermined residual amount of inhalable therapy remaining in the inhalable therapy device 106. In some embodiments, the control unit 100 can control the inhalable therapy device 106 to pause or delay delivery of the inhalable therapy. In these embodiments, the control unit 100 can control the inhalable therapy device 106 to reengage in delivering the inhalable therapy 106 when one or more predetermined or preset conditions are achieved.

At block 510, the subject 104 is preparing to sleep. At block 512, the inhalable therapy may be prepared in an inhalable therapy device 106. At block 514, the inhalable therapy device 106 may be connected to (for example, docked) at the respiratory assist device 102 according to this example embodiment. At block 516, the control unit 100 may determine when to automatically trigger delivery of the inhalable therapy to the subject 104 and set parameters for this. In particular, the control unit 100 can set a predefined length of time following the detected time at which delivery of the inhalable therapy to the subject 104 is manually triggered to subsequently use in controlling the inhalable therapy device 106 to automatically trigger delivery of the inhalable therapy to the subject 104. At block 518, the inhalable therapy device 106 is attached to the subject 104, such that the inhalable therapy can be provided to the subject 104 via their respiratory system when the subject has fallen asleep. According to this example embodiment, the inhalable therapy device 106 is attached to the subject 104 indirectly, such that the inhalable therapy can be provided to the subject 104 via their respiratory system when the subject has fallen asleep. In the embodiment illustrated in Fig. 2, for example, the inhalable therapy device 106 may deliver the inhalable therapy indirectly to the subject 104 via an interface 102c of a respiratory assist device 102. However, it will be appreciated that in other embodiments, the inhalable therapy device 106 may itself comprise such an interface to deliver the inhalable therapy directly to the subject 104.

At block 520, the control unit 100 detects that the subject 104 has fallen asleep. For example, the control unit 100 detects a sleep onset of the subject 104. At block 522, the control unit 100 may detect a predefined (or appropriate) sleep stage of the subject 104. For example, the predefined sleep stage may be the rapid eye movement (REM) sleep stage. At block 524, the control unit 100 controls the inhalable therapy device 106 to automatically trigger (or start) delivery of the inhalable therapy to the subject 104. Thus, on detecting that the subject 104 has fallen asleep, the control unit 100 controls the inhalable therapy device 106 to automatically trigger delivery of the inhalable therapy to the subject 104 at least a predefined length of time following the detected time at which delivery of the inhalable therapy to the subject 104 is manually triggered or, more specifically, at a time when the subject 104 is detected to be in the predefined sleep stage after expiry of the predefined length of time according to the illustrated example embodiment of Fig. 5. In this way, the inhalable therapy is delivered to the subject 104 at block 526.

At block 528, the inhalable therapy device 106 may detect a low level of inhalable therapy and automatically stop delivering the inhalable therapy to the subject 104. Alternatively, at block 528, the control unit 100 may detect a low level of inhalable therapy in the inhalable therapy device 106 and control the inhalable therapy device 106 to stop delivering the inhalable therapy to the subject 104. In some embodiments, the control unit 100 may determine a prescribed time of administration of inhalable therapy in order to turn off the inhalable therapy device 106 after the prescribed time. This may leave a predetermined residual amount of inhalable therapy remaining in the inhalable therapy device 106. In some embodiments, the control unit 100 can control the inhalable therapy device 106 to pause or delay delivery of the inhalable therapy. In these embodiments, the control unit 100 can control the inhalable therapy device 106 to reengage in delivering the inhalable therapy 106 when one or more predetermined or preset conditions are achieved. At block 530, the subject 104 wakes from their sleep cycle. At block 532, the inhalable therapy device 106 (and, in embodiments where the inhalable therapy is delivered via the respiratory assist device 102, also the respiratory assist device 102) is disconnected from the subject 104, such that maintenance or administration can be performed while the subject 104 is awake. The method illustrated in Fig. 5 may then be repeated.

In any of the embodiments described herein, the control unit 100 may be configured to control the inhalable therapy device 106 to automatically trigger delivery of the inhalable therapy to the subject 104 at predefined time intervals that begin at least the predefined length of time following the detected time at which delivery of the inhalable therapy to the subject 104 is manually triggered. In some embodiments where the inhalable therapy is a medication (such as a drug), the control unit 100 may be configured to control the inhalable therapy device 106 to deliver a varying dose of the medication at one or more of the times that (or each time) the inhalable therapy device 106 is automatically triggered to deliver the inhalable therapy.

In any embodiments described herein, the control unit 100 may be configured to control the inhalable therapy device 106 to further automatically trigger delivery of the inhalable therapy to the subject 104 at a predefined time prior to any one or more of an estimated wake up time for the subject 104. For example, in some embodiments, an ideal wake up time for the subject 104 may be set (e.g. by the subject themselves, a medical professional such as a doctor, a caregiver, a family member, or any other user) and the control unit 100 can be programmed to further automatically trigger delivery of the inhalable therapy at a predefined time (e.g. 2 hours) prior to that set wake up time.

Alternatively or in addition, in any embodiments described herein, the control unit 100 may be configured to control the inhalable therapy device 106 to further automatically trigger delivery of the inhalable therapy to the subject 104 at an estimated time for an onset of one or more symptoms of the subject 104. For example, where symptoms are a problem for the subject at night, a time at which symptoms usually occur during the night may be set (e.g. by the subject themselves, a medical professional such as a doctor, a caregiver, a family member, or any other user) and the control unit 100 can be programmed to further automatically trigger delivery of the inhalable therapy at that set time during the night or at a predefined time (e.g. 2 hours) prior to that set time during the night. This can further help to relieve night symptoms and/or help to improve morning symptoms.

Although the control unit 100 has been described herein with reference to Figs. 1 and 2, it will be appreciated that Figs. 1 and 2 only show the components required to illustrate certain embodiments and, in a practical implementation, the control unit 100 may be configured to communicate with and/or connect to other components or additional components to those shown.

For example, although not illustrated in Figs. 1 or 2, the control unit 100 may be configured to communicate with and/or connect to a communications interface (or communications circuitry). The communications interface can be for enabling the control unit 100 to communicate with and/or connect to one or more devices, components, interfaces, memories, or sensors. For example, the communications interface can be for enabling the control unit 100 to communicate with and/or connect to one or more of the respiratory assist device 102, the inhalable therapy device 106, one or more physiological characteristic sensors, one or more eye movement sensors, or any other device, component, interface, memory, or sensor, or any combination thereof. Thus, the control unit 100 may control the inhalable therapy device 106 via the communications interface. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications.

Although also not illustrated in Figs. 1 or 2, the control unit 100 may be configured to communicate with and/or connect to a memory. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). The memory can be configured to store program code that can be executed by the control unit 100 to cause the control unit 100 to operate in the manner described herein. Alternatively or in addition, the memory can be configured to store information acquired by the control unit 100. The control unit 100 may be configured to control the memory to store such information. For example, the control unit 100 may be configured to control the memory to store the detected time at which delivery of the inhalable therapy to the subject 104 is manually triggered, the detected time at which the subject fell asleep, the time at which the inhalable therapy device 106 is controlled to automatically trigger delivery of the inhalable therapy to the subject 104, any acquired physiological characteristic data, any acquired eye movement data, any acquired breathing pattern of the subject, or any other information, or any combination of information resulting from the method described herein.

Although also not illustrated in Figs. 1 or 2, the control unit 100 may be configured to communicate with and/or connect to a user interface. The user interface can be configured to render (or output, display, or provide) information resulting from the method described herein, such as any of the information mentioned earlier in respect of the memory. The control unit 100 may be configured to control the user interface to render such information. Alternatively or in addition, the user interface can be configured to receive a user input. For example, the user interface may allow a user to manually enter information or instructions, interact with and/or control the control unit 100. The user can be the subject themselves, a medical professional (such as a doctor), a caregiver, a family member, or any other user. The control unit 100 may be configured to acquire the user input from the user interface. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although also not illustrated in Figs. 1 or 2, the control unit 100 may comprise a battery or other power supply for powering the control unit 100 or means for connecting the control unit 100 to a mains power supply, or any other component, or any combination of components.

In addition to the control unit 100 and method 300 described herein, there is also provided a computer program product comprising a computer readable medium. The computer readable medium has computer readable code embodied therein. The computer readable code is configured such that, on execution by a suitable computer or processor, the computer or processor is caused to perform the method described. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the relevant method.

The control unit 100, method 300 and computer program product described herein can be useful in delivering an inhalable therapy to a subject, for example, to prevent or control their symptoms. For example, the control unit 100, method 300 and computer program product described herein can be useful for subjects with chronic obstructive pulmonary disease (COPD), asthma, cancer, diabetes, cardiac (or heart) failure, hypertension, stroke, or any other illness or disease that requires a therapy, which it is possible to deliver via the respiratory system of the subject. The control unit 100, method 300 and computer program product described herein can be used for the delivery of respiratory medications (such as bronchodilators, muscarinic agents, albuterol/ipratropium bromide, prednisone, theophylline, etc) in the case of COPD or asthma, the delivery of cancer treating drugs (such as 5-fluorouracil 5-FU) in the case of cancer, the delivery of insulin in the case of diabetes (for example, to help prevent overnight glucose spikes), the delivery of a lasix in the case of cardiac failure (for example, to help reduce edema), the delivery of hypertension medications (such as angiotensin-converting-enzyme ACE inhibitors) in the case of hypertension, and the delivery of stroke preventative medications (such as tissue plasminogen activators tPAs) in the case of subject at high risk of a stroke.

The control unit 100 described herein is for use with a respiratory assist device 102 and can thus be useful to provide an inhalable therapy in combination with respiratory assistance. For example, subjects with chronic obstructive pulmonary disease (COPD) often have comorbid obstructive sleep apnea (OSA) or require support via a respiratory assist device such as an oxygen cannula or ventilator. Through use of the control unit 100 described herein, a subject 104 can benefit from unobtrusive inhalable therapy delivery at night. Moreover, the control unit 100 described herein is configured to deliver an inhalable therapy at optimal times when the subject 104 is asleep. In this way, the sleep quality of the subject 104 can be improved since the inhalable therapy can reduce symptoms (such as the closure of airways or any other symptoms), which can otherwise interfere with quality sleep, and thus the quality of life of the subject 104 can be improved.

There is thus provided herein an improved manner in which to deliver an inhalable therapy to a subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A control unit (100) for use with a respiratory assist device (102) that assists a subject (104) with respiration, the control unit (100) configured to:
detect a time at which delivery of an inhalable therapy to the subject (104) is manually triggered on an inhalable therapy device (106), said inhalable therapy device comprising a nebulizer, a single dose inhaler, or a metered dose inhaler;
detect whether the subject (104) has fallen asleep; and
on detecting that the subject (104) has fallen asleep, control the inhalable therapy device (106) to automatically trigger delivery of the inhalable therapy to the subject (104) at least a predefined length of time following the detected time at which delivery of the inhalable therapy to the subject (104) is manually triggered.

2. A control unit (100) as claimed in claim 1, wherein the control unit (100) is configured to:
control the inhalable therapy device (106) to automatically trigger delivery of the inhalable therapy to the subject (104) provided that the subject (104) is detected to still be asleep.

3. A control unit (100) as claimed in claim 1 or 2, wherein the control unit (100) is configured to:
control the inhalable therapy device (106) to automatically trigger delivery of the inhalable therapy to the subject (104) on expiry of the predefined length of time.

4. A control unit (100) as claimed in claim 3, wherein the control unit (100) is configured to:
detect a sleep stage of the subject (104); and
control the inhalable therapy device (106) to automatically trigger delivery of the inhalable therapy to the subject (104) on expiry of the predefined length of time provided that the subject (104) is detected to be in a predefined sleep stage.

5. A control unit (100) as claimed in claim 1 or 2, wherein the control unit (100) is further configured to:
detect a sleep stage of the subject (104); and
control the inhalable therapy device (106) to automatically trigger delivery of the inhalable therapy to the subject (104) at a time when the subject (104) is detected to be in a predefined sleep stage after expiry of the predefined length of time.

6. A control unit (100) as claimed in claim 4 or 5, wherein the control unit (100) is configured to:
detect the sleep stage of the subject (104) from physiological characteristic data and/or eye movement data acquired from the subject (104).

7. A control unit (100) as claimed in any one of claims 4, 5 or 6, wherein the predefined sleep stage is a rapid eye movement sleep stage.

8. A control unit (100) as claimed in any one of the preceding claims, wherein the control unit (100) is configured to:
detect whether the subject (104) has fallen asleep based on a breathing pattern of the subject (104) detected by the respiratory assist device (102).

9. A control unit (100) as claimed in any one of the preceding claims, wherein the control unit (100) is configured to:
control the inhalable therapy device (106) to automatically trigger delivery of the inhalable therapy to the subject (104) at predefined time intervals that begin at least the predefined length of time following the detected time at which delivery of the inhalable therapy to the subject (104) is manually triggered.

10. A control unit (100) as claimed in any one of the preceding claims, wherein the control unit (100) is configured to:
control the inhalable therapy device (106) to further automatically trigger delivery of the inhalable therapy to the subject (104) at a predefined time prior to any one or more of:
an estimated wake up time for the subject (104); and
an estimated time for an onset of one or more symptoms of the subject (104).

11. A control unit (100) as claimed in any one of the preceding claims, wherein the inhalable therapy comprises any one or more of a medicament, a saline therapy, and a humidity therapy.

12. A control unit (100) as claimed in any one of the preceding claims, wherein the respiratory assist device (102) comprises:
a continuous positive airway pressure device;
a non-invasive ventilation device; or
an oxygen delivery device.

## Patentansprüche

1. Steuereinheit (100) zur Verwendung mit einem Beatmungsgerät (102), welches einen Patienten (104) bei der Atmung unterstützt, wobei die Steuereinheit (100) für Folgendes konfiguriert ist: Erfassen eines Zeitpunkts, zu dem die Abgabe einer inhalierbaren Therapie an den Patienten (104) manuell an einem Gerät für die inhalierbare Therapie (106) ausgelöst wird, wobei das Gerät für die inhalierbare Therapie einen Vernebler, einen Einzeldosis-Inhalator oder einen Dosier-Inhalator umfasst; Erfassen, ob der Patient (104) eingeschlafen ist; und bei der Feststellung, dass die Person (104) eingeschlafen ist, das Gerät für die inhalierbare Therapie (106) so zu steuern, dass die Abgabe der inhalierbaren Therapie an die Person (104) mindestens eine vordefinierte Zeitspanne nach dem festgestellten Zeitpunkt, zu dem die Abgabe der inhalierbaren Therapie an die Person (104) manuell ausgelöst wird, automatisch ausgelöst wird.

2. Steuereinheit (100) nach Anspruch 1, wobei die Steuereinheit (100) konfiguriert ist, um:
das Gerät für die inhalierbare Therapie (106) so zu steuern, dass die Abgabe der inhalierbaren Therapie an die Person (104) automatisch ausgelöst wird, sofern festgestellt wird, dass die Person (104) noch schläft.

3. Steuereinheit (100) nach Anspruch 1 oder 2, wobei die Steuereinheit (100) konfiguriert ist, um:
das Gerät für die inhalierbare Therapie (106) so zu steuern, die Abgabe der inhalierbaren Therapie an die Person (104) nach Ablauf der vordefinierten Zeitspanne automatisch auszulösen.

4. Steuereinheit (100) nach Anspruch 3, wobei die Steuereinheit (100) konfiguriert ist, um:
eine Schlafphase des Patienten (104) zu erfassen; und
das Gerät für die inhalierbare Therapie (106) so zu steuern, dass die Abgabe der inhalierbaren Therapie an den Patienten (104) nach Ablauf der vordefinierten Zeitspanne automatisch ausgelöst wird, vorausgesetzt, es wird erfasst, dass sich der Patient (104) in einer vordefinierten Schlafphase befindet.

5. Steuereinheit (100) nach Anspruch 1 oder 2, wobei die Steuereinheit (100) konfiguriert ist, um:
ein Schlafphase des Patienten (104) zu erfassen; und
das Gerät für die inhalierbare Therapie (106) so zu steuern, dass die Abgabe der inhalierbaren Therapie an den Patienten (104) zu einem Zeitpunkt automatisch auslöst, zu dem erfasst wird, dass sich die Person (104) nach Ablauf der vordefinierten Zeitspanne in einer vordefinierten Schlafphase befindet.

6. Steuereinheit (100) nach Anspruch 4 oder 5, wobei die Steuereinheit (100) konfiguriert ist, um:
die Schlafphase des Patienten (104) anhand von physiologischen Merkmalsdaten und/oder Augenbewegungsdaten, die von dem Patienten (104) erfasst wurden, zu erkennen.

7. Steuereinheit (100) nach einem der Ansprüche 4, 5 oder 6, wobei die vordefinierte Schlafphase eine Rapid-Eye-Movement-Schlafphase ist.

8. Steuereinheit (100) nach einem der vorhergehenden Ansprüche, wobei die Steuereinheit (100) konfiguriert ist zum: Erfassen, ob das Subjekt (104) eingeschlafen ist, basierend auf einem Atemmuster des Subjekts (104) von der Atmungsunterstützungsvorrichtung (102) erkannt.

9. Steuereinheit (100) nach einem der vorhergehenden Ansprüche, wobei die Steuereinheit (100) so konfiguriert ist, dass sie: das Gerät für die inhalierbare Therapie (106) so steuert, dass es die Abgabe der inhalierbaren Therapie an den Patienten (104) in vordefinierten Zeitintervallen automatisch auslöst, die mindestens in der vordefinierten Zeitspanne nach dem erfassten Zeitpunkt beginnen, zu dem die Abgabe der inhalierbaren Therapie an den Patienten (104) manuell ausgelöst wurde.

10. Steuereinheit (100) nach einem der vorhergehenden Ansprüche, wobei die Steuereinheit (100) so konfiguriert ist, dass sie: das Gerät für die inhalierbare Therapie (106) zum weiteren automatischen Auslösen der Abgabe der inhalierbaren Therapie an den Patienten (104) zu einem vordefinierten Zeitpunkt vor einem oder mehreren der folgenden Punkte steuert: einer geschätzten Aufwachzeit für den Patienten (104); und einem geschätzten Zeitpunkt für das Auftreten eines oder mehrerer Symptome des Patienten (104).

11. Steuereinheit (100) nach einem der vorhergehenden Ansprüche, wobei die inhalierbare Therapie eines oder mehrere der folgenden Elemente umfasst: ein Medikament, eine Kochsalztherapie und eine Feuchtigkeitstherapie.

12. Steuereinheit (100) nach einem der vorhergehenden Ansprüche, wobei das Beatmungsgerät (102) Folgendes umfasst: ein Gerät zur kontinuierlichen positiven Beatmung; ein Gerät zur nicht-invasiven Beatmung; oder ein Gerät zur Sauerstoffzufuhr.

## Revendications

1. Unité de commande (100) destinée à être utilisée avec un dispositif d'assistance respiratoire (102) qui assiste un sujet (104) dans sa respiration, l'unité de commande (100) étant configurée pour:
détecter un moment auquel la délivrance d'une thérapie inhalable au sujet (104) est déclenchée manuellement sur un dispositif de thérapie inhalable (106), ledit dispositif de thérapie inhalable comprenant un nébuliseur, un inhalateur à dose unique ou un inhalateur à dose mesurée;
détecter si le sujet (104) s'est endormi; et
en détectant que le sujet (104) s'est endormi, commander le dispositif de thérapie inhalable (106) pour déclencher automatiquement la délivrance de la thérapie inhalable au sujet (104) au moins pendant une durée prédéfinie après le moment détecté auquel la délivrance de la thérapie inhalable au sujet (104) est déclenchée manuellement.

2. Unité de commande (100) selon la revendication 1, dans laquelle l'unité de commande (100) est configurée pour:
commander le dispositif de thérapie inhalable (106) pour déclencher automatiquement la délivrance de la thérapie inhalable au sujet (104) à condition que le sujet (104) soit détecté comme étant toujours endormi.

3. Unité de commande (100) selon la revendication 1 ou 2, dans laquelle l'unité de commande (100) est configurée pour:
commander le dispositif de thérapie inhalable (106) pour déclencher automatiquement la délivrance de la thérapie inhalable au sujet (104) à l'expiration de la durée prédéfinie.

4. Unité de commande (100) selon la revendication 3, dans laquelle l'unité de commande (100) est configurée pour:
détecter un stade de sommeil du sujet (104); et
commander le dispositif de thérapie inhalable (106) pour déclencher automatiquement la délivrance de la thérapie inhalable au sujet (104) à l'expiration de la durée prédéfinie, à condition que le sujet (104) soit détecté comme étant dans un stade de sommeil prédéfini.

5. Unité de commande (100) selon la revendication 1 ou 2, dans laquelle l'unité de commande (100) est en outre configurée pour:
détecter un stade de sommeil du sujet (104); et
commander le dispositif de thérapie inhalable (106) pour déclencher automatiquement la délivrance de la thérapie inhalable au sujet (104) à un moment où le sujet (104) est détecté comme étant dans un stade de sommeil prédéfini après l'expiration de la durée prédéfinie.

6. Unité de commande (100) selon la revendication 4 ou 5, dans laquelle l'unité de commande (100) est configurée pour:
détecter le stade de sommeil du sujet (104) à partir de données de caractéristiques physiologiques et/ou de données de mouvements oculaires acquises auprès du sujet (104).

7. Unité de commande (100) selon l'une quelconque des revendications 4, 5 ou 6, dans laquelle le stade de sommeil prédéfini est un stade de sommeil à mouvement oculaire rapide.

8. Unité de commande (100) selon l'une quelconque des revendications précédentes, dans laquelle l'unité de commande (100) est configurée pour:
détecter si le sujet (104) s'est endormi sur la base d'un modèle de respiration du sujet (104) détecté par le dispositif d'assistance respiratoire (102).

9. Unité de commande (100) selon l'une quelconque des revendications précédentes, dans laquelle l'unité de commande (100) est configurée pour:
commander le dispositif de thérapie inhalable (106) pour déclencher automatiquement la délivrance de la thérapie inhalable au sujet (104) à des intervalles de temps prédéfinis qui commencent au moins la durée prédéfinie suivant le moment détecté auquel la délivrance de la thérapie inhalable au sujet (104) est déclenchée manuellement.

10. Unité de commande (100) selon l'une quelconque des revendications précédentes, dans laquelle l'unité de commande (100) est configurée pour:
commander le dispositif de thérapie inhalable (106) pour déclencher en outre automatiquement la délivrance de la thérapie inhalable au sujet (104) à un moment prédéfini avant l'un ou plusieurs des éléments suivants:
une heure de réveil estimée pour le sujet (104); et
un moment estimé pour l'apparition d'un ou plusieurs symptômes du sujet (104).

11. Unité de commande (100) selon l'une quelconque des revendications précédentes, dans laquelle la thérapie inhalable comprend un ou plusieurs médicaments, une thérapie saline et une thérapie par l'humidité.

12. Unité de commande (100) selon l'une quelconque des revendications précédentes, dans laquelle le dispositif d'assistance respiratoire (102) comprend:
un dispositif de pression positive continue des voies aériennes;
un dispositif de ventilation non invasive; ou
un dispositif d'administration d'oxygène.
